# EUROPEAN PATENT APPLICATION

(11) **EP 2 319 914 A1**
(43) Date of publication of application: **11.05.2011**
(21) Application number: 09425451.3
(22) Date of filing: 09.11.2009
(51) Int. Cl.: C12N 5/04, A61K 36/00

(54) **Preparation and use of plant meristem cells with a high content of caffeic acid derivatives**

(71) Applicant: I.R.B. Istituto Di Ricerche Biotecnologiche S.r.l., 36077 Altavilla Vicentina (VI) (IT)
(72) Inventor: Sgaravatti, Elena, 36077 Altavilla Vicentina (Vicenza) (IT); Dal Toso, Roberto, 36077 Altavilla Vicentina (Vicenza) (IT); Pressi, Giovanna, 36077 Altavilla Vicentina (Vicenza) (IT)
(74) Representative: Long, Giorgio

(57) **Abstract**

The present invention refers to the preparation of plant cell cultures and to their use in the cosmetic, nutritional, zootechnical, and pharmaceutical fields.

In particular, the present invention refers to a preparation of meristem cells with a high content of caffeic acid derivatives, said cells being derived from cell cultures belonging to the genera *Achillea*, *Ajuga, Buddleja, Centella, Cynaris, Echinacea, Forsythia, Gardenia, Glycyrrhiza, Hippeastrum, Hoodia, Lamium, Leontopodium, Lippia, Lonicera, Magnolia, Marrubium, Passiflora, Phytolacca, Plantago, Ruta, Stachys, Stapelia, Syringa, Teucriun,* or *Zanthoxylum.*

## Description

### Field of the invention

The present invention refers to the preparation of plant cell cultures and to their use in the cosmetic, nutritional, zootechnical, and pharmaceutical fields.

### State of the art

Caffeic acid derivatives are organic compounds of plant origin belonging to the wide family of secondary metabolites. These bioactive plant compounds show multiple functions: defense against herbivores and against microbial attacks, protection of plants from UV light, and action as molecular signals. Biological activities of caffeic acid derivatives have been largely described in the literature. In particular, these compounds are involved in the protection of proteins, lipids, and nucleic acids from free radical-induced damages (Gebhardt R. et al, 1997, Toxicol. Appl. Pharm. 144:279-286; Perez Garcia F. et al, 2000, Free Radical Res., 33: 661-665); they inhibit cholesterol biosynthesis, contribute to the prevention of atherosclerosis and vascular disorders (Brown J.E. Et al, 1998, Free Radical Res., 29:247-255; Kraft K., 1997, 4: 369-378; Pittlern MH et al, Perfusion, 11:338-340), have hepatoprotective, coleretic, and diuretic activities (Dogan S. et al, 2005, J. Agric. Food Chem., 53:776-785), antiviral activity against HIV (Mcdougall B. et al, 1998, Antimicrob. Agents Ch., 42: 140-146; Shanina J. et al, 2001, Tetrahedron Lett., 42:3383-3385), antibacterial and antifungal activities (Martinov et al, 1999, Acta Horticulturae, 501:111-114; Zhu XF, 2005, Fitoterapia; 76: 108-111), anti-inflammatory and cicatrizant activities (Korkina et al., 2007, Cellular and Molecular Biology 53(5): 78-83), and potential tumor-preventive activity (Kurata R. et al, 2007, J. Agric. Food Chem. 55 (1) :185-190) .

The presence of caffeic acid derivatives in plant tissues is subject to wide fluctuations depending on geographical origin, season variability, and plant contamination with parasites. Furthermore, the content of these compounds in plants is always very limited.

### Summary of the invention

It has been found by the inventors of the present invention that using stabilized and selected plant cell cultures is an advantageous alternative to obtain meristem cells with a high content of caffeic acid derivatives.

Indeed, plant cell culture technology permits to overcome the problems mentioned above.

Hence, an object of the invention is a process for the preparation, also on an industrial basis, of meristem cells with a high content of caffeic acid derivatives, from cell cultures of plants belonging to the genera *Achillea, Ajuga, Buddleja, Centella, Cynaris, Echinacea, Forsythia, Gardenia, Glycyrrhiza, Hippeastrum, Hoodia, Lamium, Leontopodium, Lippia, Lonicera, Magnolia, Marrubium, Passiflora, Phytolacca, Plantago, Ruta, Stachys, Stapelia, Syringa, Teucriun,* and *Zanthoxylum,* and in particular to the species *Achillea millefolium, Ajuga reptans, Buddleja davidii, Centella asiatica, Cynaris scolimus, Echinacea angustifolia, Echinacea purpurea, Echinacea pallida, Forsythia viridissima, Gardenia jasminoides, Glycyrrhiza glabra, Glycyrrhiza echinata, Hippeastrum lady jane, Hoodia gordonii, Lamium album, Leontopodium alpinun, Lippia citriodora, Lonicera japonica, Magnolia grandiflora, Magnolia solangeniana, Marrubium vulgare, Passiflora palmeri, Passiflora quadrangularis, Phytolacca americana, Plantago lanceolata, Ruta patavina, Scrophularia nodosa, Stachys officinalis, Stapelia grandiflora, Syringa vulgaris, Teucriun chanaedrys,* and *Zanthoxylum americanum.*

It has been observed that meristem cells obtained from the plant species listed above show, as such, high antioxidant and anti-inflammatory activities, inhibitory activity in relation to the enzymes collagenase, hyaluronidase, and 5-alpha reductase, collagen production inducing activity, photoprotective, depigmenting, antiviral, antimicrobic, and antiaging activities.

Hence, a preparation of meristem cells obtained as described below represents a further object of the present invention, said preparation being preferably a glycerol suspension or solution, a freeze-dried or spray-dried preparation.

Other objects of the invention are said preparations of meristem cells for use in cosmetic, nutritional, zootechnical, and pharmaceutical applications.

Further objects and advantages of the present invention will become more apparent from the following detailed description of the invention.

### Brief description of the figures

Fig. 1: antioxidant activity of glycerolic stems from *Buddleja davidii* (BDSG);
Fig. 2: hyaluronidase inhibitory activity of freeze-dried stems from *Leontopodium alpinum* (LAS);
Fig. 3: inhibitory activity in relation to NO production of spray-dried stems from *Lippia citriodora IRB Lippia C19* (LCS);
Fig. 4: collagenase inhibitory activity of freeze-dried stems from *Gardenia jasminoides* (GjS);
Fig. 5: collagen stimulatory activity of freeze-dried stems from *Echinacea angustifolia IRBEA66* (Eas).

### Detailed description of the invention

The main caffeic acid derivatives isolated from cell cultures of the plant species listed previously are reported in the following Table 1.

**Table 1.**

| | **CELL LINE** | **Main phenylpropanoids isolated from the culture** | **Main caffeoyilquinic acids isolated from the culture** |
|---|---|---|---|
| **1.** | ***ACHILLEA MILLEFOLIUM*** | | 3-0-caffeoylquinic acid |
| **2.** | ***AJUGA REPTANS*** | Teupolioside, isoteupolioside, and verbascoside | |
| **3.** | ***BUDDLEJA DAVIDII*** | Verbascoside | |
| **4.** | ***CENTELLA ASIATICA*** | | 3,5-dicaffeoyl-4- malonylquinic acid |
| **5.** | ***CYNARIS SCOLYMUS*** | | 1,3-dicaffeoylquinic acid, 1,5-dicaffeoylquinic acid, 3,5-dicaffeoylquinic acid |
| **6.** | ***ECHINACEA ANGUSTIFOLIA*** | Echinacoside | |
| **7.** | ***ECHINACEA PALLIDA*** | | 3-0-caffeoylquinic acid |
| **8.** | ***ECHINACEA PURPUREA*** | | 1,5-dicaffeoylquinic acid, 3,5-dicaffeoylquinic acid |
| **9.** | ***FORSYTHIA VIRIDISSIMA*** | Verbascoside, forsythoside, isoverbascoside, campneoside II, and echinacoside | |
| **10.** | ***GARDENIA JASMINOIDES*** | 4-0-beta-D-glucopyranosyl caffeic acid | |
| **11.** | ***GLYCYRRHIZA GLABRA*** | | 3-0-caffeoylquinic acid |
| **12.** | ***GLYCYRRHIZA ECHINATA*** | | 3-0-caffeoylquinic acid |
| **13.** | ***HIPPEASTRUM LADY JANE*** | | 3-0-caffeoylquinic acid |
| **14.** | ***HOODIA GORDONII*** | | 3-0-caffeoylquinic acid |
| **15.** | ***LAMIUM ALBUM*** | Verbascoside, isoverbascoside | |
| **16.** | ***LEONTOPODIUM ALPINUM*** | | 1,5-dicaffeoylquinic acid, 3,5-dicaffeoylquinic acid, 3- 0-caffeoylquinic acid |
| **17.** | ***LIPPIA CITRODORA*** | Verbascoside | |
| **18.** | ***LONICERA JAPONICA*** | | 3-O-caffeoylquinic acid |
| **19.** | ***MAGNOLIA GRANDIFLORA*** | Echinacoside | |
| **20.** | ***MAGNOLIA SOLANGENIANA*** | | 3-O-caffeoylquinic acid |
| **21.** | ***MARRUBIUM VULGARE*** | Forsythoside, verbascoside, and campneoside | |
| **22.** | ***PASSIFLORA PALMERI*** | 4-0-beta-D-glucopyranosyl caffeic acid | |
| **23.** | ***PASSIFLORA QUADRANGULARIS*** | 4-0-beta-D-glucopyranosyl caffeic acid | |
| **24.** | ***PHYTOLACCA AMERICANA*** | | 3-0-caffeoylquinic acid |
| **25.** | ***PLANTAGO LANCEOLATA*** | Plantamajoside | |
| **26**. | *RUTA PATAVINA* | | 3-0-caffeoylquinic acid |
| **27.** | ***SCROPHULARIA NODOSA*** | Verbascoside and isoverbascoside | |
| **28.** | ***STACHYS OFFICINALIS*** | Verbascoside, forsythoside, isoverbascoside, echinacoside, and campneoside | |
| **29.** | ***STAPELIA GRANDIFLORA*** | | 3-0-caffeoylquinic acid |
| **30.** | ***SYRINGA VULGARIS*** | Verbascoside, isoverbascoside | |
| **31.** | ***TEUCRIUN CHANAEDRYS*** | Teupolioside | |
| **32.** | ***ZANTHOXYLUM AMERICANUM*** | 2'-O-caffeoyl-D-glucopyranoside ester | |

The process to prepare plant meristem cells with a high content of caffeic acid derivatives, also in industrial quantities, comprises the following stages in sequence:
1) selection of clones deriving from cell cultures of plants belonging to the genera *Achillea, Ajuga, Buddleja, Centella, Cynaris, Echinacea, Forsythia, Gardenia, Glycyrrhiza, Hippeastrum, Hoodia, Lamium, Leontopodium, Lippia, Lonicera, Magnolia, Marrubium, Passiflora, Phytolacca, Plantago, Ruta, Stachys, Stapelia, Syringa, Teucriun,* and *Zanthoxylum,* in which maximized quantities of caffeic acid derivatives are present;
2) recovery of the plant biomass deriving from a cell culture of said clones selected on the basis of stage 1) on a liquid culture medium
3) separation of cells from the liquid culture medium
4) optionally, freeze-drying and/or spray-drying of said cells or solution or suspension of said cells in glycerol.

Preferably, the biomass recovery stage occurs after growing the cells deriving from the species listed above in a fermenter, comprising a first stage of selection of cell cultures based on the proliferation rate and on the content of caffeic acid derivatives.

In an embodiment, this process envisages the collection of tissues (preferably leaves, stems, and buds) from plants selected from one or more of said genera, preferably from the following species: *Achillea millefolium, Ajuga reptans, Buddleja davidii, Centella asiatica, Cynaris scolimus, Echinacea angustifolia, Echinacea purperea, Echinacea pallida, Forsythia viridissima, Gardenia jasminoides, Glycyrrhiza glabra, Glycyrrhiza echinata, Hippeastrum lady jane, Hoodia gordonii, Lamium album, Leontopodium alpinun, Lippia citriodora, Lonicera japonica, Magnolia grandiflora, Magnolia solangeniana, Marrubium vulgare, Passiflora palmeri, Passiflora quadrangularis, Phytolacca americana, Plantago lanceolata, Ruta patavina, Scrophularia nodosa, Stachys officinalis, Stapelia grandiflora, Syringa vulgaris, Teucriun chanaedrys,* and *Zanthoxylum americanum,* the cleaning thereof, for example under running water, cutting into fragments of 2-5 cm, and sterilization on plates, for example by sequential treatment with 70% ethanol for approximately 15 minutes, 2% sodium hypochlorite for approximately 5 minutes, and finally 0.05% HgC1₂ for approximately 1 minute. Between treatments, the plant fragments are washed, typically three or more times, with sterile distilled water.

Each fragment of said tissue, chopped-up even further (explants), is placed on a Petri dish containing a nutrient medium, solidified by the addition of agar and supplemented with growth hormones without the addition of any antibiotics. The number of explants performed influences the outcome of the subsequent stages. Generally, from 2000 to 5000 uncontaminated explants are sufficient to proceed to the subsequent selection stage.

After a suitable period of time, an undifferentiated callus tissue forms, which is then multiplied after a transfer onto a greater surface area with fresh medium.

Furthermore, the plant cell culture derived from the undifferentiated callus tissue is stabilized preferably by means of a certain number of transfers (sub-cultures) onto fresh culture medium. Particularly, it has been observed that in order to obtain a stable cell culture, it is important to perform at least ten sub-cultures. Such medium is solid in nature and may be advantageously constituted by 0.8-1% agar in a standard culture medium, to which plant peptone has been added, allowing a balanced supply of aminoacids and guaranteeing the maintenance of good cell wall integrity. Preferably, plant peptone will be added in quantities ranging between 500 and 4000 mg/1 of culture medium.

A "stable cell culture" is defined as a culture having the following characteristics:
- high and constant proliferation rate over time;
- preservation of the same phenotypic characteristics (cell colour, aggregate friability, size etc.) throughout various sub-cultures;
- constant content of caffeic acid derivatives per unit of mass, over the course of the various sub-cultures (the content of the caffeic acid derivatives is assessed by chemical analysis of the extracts);
- constant primary metabolite content (proteins, lipids, and polysaccharides) per unit of mass.

Following the stabilization stage, the cell culture is subjected to "clonal selection". Such selection consists of growing the stabilized cells for an appropriate amount of time (typically, 10-15 days of culture). Subsequently, individual cell aggregates are taken from the solid culture medium and each of said cell aggregates is inoculated into the liquid culture medium described above.

Following fermentation for a sufficient period of time to obtain adequate multiplication of the cell aggregate (hereinafter referred to as "clone"), a time generally comprised of between 10 and 15 days, the content of the metabolite of interest is determined for each clone.

Such operations may be repeated until a plant cell culture clone is selected, in which production of the caffeic acid derivatives is the highest.

It should be remembered that alternating periods of culture on solid and liquid media is essential for the clonal selection process of the present invention. Hence, it is essential that the clonal selection process described above does not conclude with the identification of the most active clone, but is constantly repeated so as to keep the selected clone phenotypically homogeneous.

The selected plant cell culture is then multiplied in order to obtain a sufficient quantity of biomass to carry out the production fermentation stage. Said quantity will depend on the specific production requirements, on the kind of plant cell culture used, and on the type of metabolite that it is desired to produce.

The biomass thus obtained may be passed directly into the final fermenter or can be subjected to one or more further growth stages in liquid medium, working with intermediate volumes.

Preferably, the process just illustrated comprises the stages of:
a) cultivating a predetermined plant cell culture, stabilized for a sufficient period of time to allow the multiplication of said cell culture to give substantially distinct cell clusters, on solid medium;
b) removing said substantially distinct cell clusters from said solid medium and placing each of them in a separate liquid culture medium;
c) cultivating each of the said substantially distinct cell clusters in said liquid culture medium for a sufficiently long period of time to allow multiplication of said cell cluster and analytical determination of the primary and/or secondary metabolites produced thereby;
d) performing a qualitative and quantitative determination of the primary and/or secondary metabolites produced by each of said cell clusters in said liquid culture medium;
e) selecting the cell cluster capable of producing the greatest quantity of said metabolite of interest;
f) repeating the process cycle according to stages a), b), c), d), and e) on said cell cluster, selected according to stage e), a sufficient number of times until the quantity of said metabolite of interest produced by a selected cell cluster, and by the cell cluster deriving from further selection cycles, is essentially constant.

In addition, the subsequent fermentation may preferably comprise the following stages:
A) inoculation of said plant clone into liquid medium and the multiplication thereof for a sufficient period of time to obtain an increase in cellular mass of at least 300% of the weight thereof;
B) optionally, transfer of the suspension obtained from stage A) into fresh liquid culture medium and the multiplication thereof for a sufficient period of time to obtain an increase in cellular mass of at least 300% of the weight thereof;
C) optionally, repetition of stage B) at least one additional time;
D) transfer of the suspension obtained in stages A), B), or C) into fresh liquid culture medium in a fermenter to give a biomass and conduct the fermentation under such conditions and for a sufficient period of time so as to obtain, within said biomass, the production of said concentration of the caffeic acid derivatives.

In accordance with one preferred embodiment, the fermentation will normally be performed at a temperature between 15°C and 35°C, typically around 25°C and for a period of time normally between 7 and 40 days, preferably between 14 and 21 days. It is essential that the biomass be adequately aerated and that at the same time be kept stirred by means of stirring external to the fermenter. Indeed, it has been observed that the plant biomass is comprised of cells having cell walls that are poorly resistant to rupture. A stirrer submerged into the biomass acts mechanically on the cells and can easily cause the lysis thereof. However, it is necessary that the stirring, although delicate, be efficient, above all in the final fermentation stages when the biomass greatly increases in density. For the purposes of the present invention, particularly appropriate means of stirring are orbital means of stirring. Such means of stirring preferably operate at 40-200 rpm, more preferably at about 120 rpm.

It is appropriate that the volume of the container (fermenter) in which the fermentation occurs be considerably greater than the biomass volume. Typically, the volume of the reactor will be from 50% to 200% greater than the biomass volume.

As already mentioned, efficient fermentation requires adequate oxygenation. Oxygenation is normally performed by using sterile air with a flow rate of 0.5-4 1/minute, more preferably 2-2.5 1/minute, for a volume of 10 litres of biomass. Alternatively, gas mixtures containing from 10% to 100% v/v of oxygen may be used.

As mentioned previously in relation to stirring, also oxygenation by means of exceedingly violent bubbling can cause rupturing of the cell walls. Hence, it is necessary to ensure that oxygenation is performed delicately, for example by bubbling through appropriate diffusers. It will be preferable to use means of air or oxygen diffusion with nozzle delivery flow speeds comprised of between 10 m/min and 600 m/min, more preferably between 50 m/min and 350 m/min.

Also the shape of the fermentation chamber has significant importance. Indeed, it is recommended that it has a smooth and uniform surface, i.e. that there are no edges, corners, or other parts which can cause the rupture of the biomass cell walls.

According to one particular embodiment of the present invention, additives increasing water oxygen solubility will be added to the biomass. Such additives will be preferably selected from those substances defined as "artificial blood", for example perfluorinated hydrocarbons (PFC).

In particular, for the purpose of the present invention, stabilized cell cultures derived from plant tissues of plants of said genera and preferably of *Achillea millefolium, Ajuga reptans, Buddleja davidii, Centella asiatica, Cynaris* scolimus, *Echinacea angustifolia, Echinacea purperea, Echinacea pallida, Forsythia viridissima, Gardenia jasminoides, Glycyrrhiza glabra, Glycyrrhiza echinata, Hippeastrum lady jane, Hoodia gordonii, Lamium album, Leontopodium alpinun, Lippia citriodora, Lonicera japonica, Magnolia grandiflora, Magnolia solangeniana, Marrubium vulgare, Passiflora palmeri, Passiflora quadrangularis, Phytolacca americana, Plantago lanceolata, Ruta patavina, Scrophularia nodosa, Stachys officinalis, Stapelia grandiflora, Syringa vulgaris, Teucriun chanaedrys,* and *Zanthoxylum americanum* were selected for their ability to produce caffeic acid derivatives in quantity greater than 0.1%, expressed as weight of fresh cells, preferably greater than 1%, and more preferably greater than 5%.

### Process for preparation of stems (meristem cells)

The process for the preparation of meristem cells from the cell cultures mentioned above envisages the following stages in sequence:
1. selection of the clone deriving from cell cultures of plants belonging to the genera mentioned above, which contains the highest concentration of caffeic acid derivatives;
2. recovery of the plant biomass deriving from a cell culture of said selected clones in liquid medium;
3. separation of the cells from the liquid culture medium;
4. optionally, freeze-drying or spray-drying of the cells or of the cell solution or suspension in glycerol or in glycerol with the addition of xanthan gum in a quantity ranging from 0.1 to 2% w/w, preferably around 0.3% w/w, or in buthylenglycol.

### A. preparation of glycerolic stems

In particular, cell cultures of cell lines from *Achillea millefolium, Ajuga reptans, Buddleja davidii, Centella asiatica, Cynaris* scolimus, *Echinacea angustifolia, Echinacea purperea, Echinacea pallida, Forsythia viridissima, Gardenia jasminoides, Glycyrrhiza glabra, Glycyrrhiza echinata, Hippeastrum lady jane, Hoodia gordonii, Lamium album, Leontopodium alpinun, Lippia citriodora, Lonicera japonica, Magnolia grandiflora, Magnolia solangeniana, Marrubium vulgare, Passiflora palmeri, Passiflora quadrangularis, Phytolacca americana, Plantago lanceolata, Ruta patavina, Scrophularia nodosa, Stachys officinalis, Stapelia grandiflora, Syringa vulgaris, Teucriun chanaedrys,* and *Zanthoxylum americanum,* stabilized and selected as mentioned above, are collected at an age between 7 and 21 days. Cells are preliminarily separated from the culture medium by gravity filtration, pressure filtration, or centrifugation through a porous mesh, for example a nylon, steel, or cotton mesh etc., with a preferred porosity ranging between 50 µm and 150 µm. Meristem cells separated from the culture medium are suspended in glycerol in a weight/volume ratio ranging between 5:95 and 50:50, preferably about 20:80. The preparations thus obtained represent the glycerolic stems.

### B. Preparation of freeze-dried stems

In accordance with a first embodiment variant of the process, cell suspension cultures of cell lines from *Achillea millefolium, Ajuga reptans, Buddleja davidii, Centella asiatica, Cynaris* scolimus, *Echinacea angustifolia, Echinacea purperea, Echinacea pallida, Forsythia viridissima, Gardenia jasminoides, Glycyrrhiza glabra, Glycyrrhiza echinata, Hippeastrum lady jane, Hoodia gordonii, Lamium album, Leontopodium alpinun, Lippia citriodora, Lonicera japonica, Magnolia grandiflora, Magnolia solangeniana, Marrubium vulgare, Passiflora palmeri, Passiflora quadrangularis, Phytolacca americana, Plantago lanceolata, Ruta patavina, Scrophularia nodosa, Stachys officinalis, Stapelia grandiflora, Syringa vulgaris, Teucriun chanaedrys,* and *Zanthoxylum americanum,* stabilized, selected, and collected at an age between 7 and 21 days, are preliminarily separated from the culture medium by gravity filtration, pressure filtration, or centrifugation through a porous mesh, for example a nylon, steel, cotton mesh etc, with a preferred porosity ranging between 50 µm and 150 µm. The obtained cells are subjected to freeze-drying according to standard protocols. The freeze-dried cells obtained represent the freeze-dried stems.

### C. Preparation of spray-dried stems

According to a further embodiment of the invention, cell suspension cultures of the cell lines mentioned above, collected at an age between 7 and 21 days and filtered according to the general procedure described in B, are subjected to a spray-drying process according to standard techniques. The powders obtained represent the spray-dried stems.

The "spray-drying" technique is a known and widely used technique in the alimentary and pharmaceutical fields. It envisages the atomization of a liquid (in the present case of an aqueous suspension of meristem cells) by appropriate nozzles in a chamber where the atomized liquid is hit (by a co-counter or a countercurrent flow) by a hot gas (air or nitrogen). This allows the instant evaporation of water present in the suspension and cells form a powder with a controlled particle size distribution (depending also on the type of spray nozzle used). Equipments for spray-drying are commercially available.

As a non-limiting example, reported here are the preparation processes of glycerolic, freeze-dried, and spray-dried cells obtained from cell cultures stabilized and selected for their high content of caffeic acid derivatives.

### EXAMPLE 1 - PREPARATION OF GLYCEROLIC STEMS FROM BUDDLEJA DAVIDII

Cells stabilized and selected as described previously, deriving from a culture of *Buddleja davidii*, grown on solid medium (Gamborg B5 containing 1% agar (w/v), 30 g/l sucrose, 1 g/l plant peptone, 1 mg/l NAA, 0.2 mg/l IAA, and 1 mg/l kinetin, final pH 6.5) were inoculated into 5 x 3 l flasks, each containing 1000 ml of liquid medium (Gamborg B5 containing 30 g/l sucrose, 1 g/l plant peptone, 1 mg/l NAA, 0.2 mg/l IAA, and 1 mg/l kinetin, final pH 6.5). The amount of plant cells inoculated into the liquid medium was equal to 5% w/v. The suspensions thus obtained were incubated at 25°C in the dark and placed on an orbital stirrer at 120 rpm. After 14 days of incubation, the plant biomass (5 liters of cell suspension) was collected and filtered through a nylon mesh with a porosity of 50 µm. The collected cells were suspended in glycerol at a 20:80 weight to volume ratio. 1200 g of cells with a content of caffeic acid derivatives equal to 2.4 g were obtained from 5 liters of cell suspension. The obtained preparation represents the glycerolic stems from *Buddleja davidii.*

### EXAMPLE 2 - PREPARATION OF FREEZE-DRIED STEMS FROM LEONTOPODIUM ALPINUM

Cells stabilized and selected as described previously, deriving from a culture of *Leontopodium alpinum,* grown on solid medium (Gamborg B5 containing 1% agar (w/v), 20 g/l sucrose, 1 g/l plant peptone, 2 mg/l NAA, 0.4 mg/l IAA, and 0.5 mg/l kinetin, final pH 6.5) were inoculated in 5 x 3 1 flasks, each containing 1000 ml of liquid medium (Gamborg B5 containing 20 g/l sucrose, 1 g/l plant peptone, 2 mg/l NAA, 0.4 mg/l IAA, and 1 mg/l kinetin, final pH 6.5). The amount of plant cells inoculated into the liquid medium was equal to 5% w/v. The suspensions thus obtained were incubated at 25°C in the dark and placed on an orbital stirrer at 120 rpm. After 14 days of incubation the plant biomass (5 liters of cell suspension) was collected and filtered through a nylon mesh with a porosity of 50 µm. The collected cells were subjected to freeze-drying. 40 g of freeze-dried cells with a content of caffeic acid derivatives of 5.6 g were obtained from 5 liters of cell suspension. The obtained preparation represents the freeze-dried stems from Leontopodium alpinum.

### EXAMPLE 3 ― PREPARATION OF SPRAY-DRIED STEMS FROM LIPPIA CITRIODORA

Cells stabilized and selected as described previously, deriving from a culture of *Lippia citriodora IRB Lippia C19* (Deposit number at DSMZ, Deutsche Sammlung von Mikroorganismen und Zellkulturen, Braunschweig, D: DSM 19511, deposit date: May 16^{th}, 2007), grown on solid medium (Gamborg B5 containing 1% agar (w/v), 30 g/l sucrose, 2 g/l plant peptone, 1 mg/l NAA, 0.2 mg/l IAA, and 1 mg/l kinetin, final pH 6.5) were inoculated in 5 x 3 1 flasks, each containing 1000 ml of liquid medium (Gamborg B5 containing 30 g/l sucrose, 2 g/l plant peptone, 1 mg/l NAA, 0.2 mg/l IAA, and 1 mg/l kinetin, final pH 6.5). The amount of plant cells inoculated into the liquid medium was equal to 5% w/v. The suspensions thus obtained were incubated at 25°C in the dark and placed on an orbital stirrer at 120 rpm. After 14 days of incubation the plant biomass (5 liters of cell suspension) was collected and filtered through a nylon mesh with a porosity of 50 µm. The collected cells were subjected to spray-drying. 52 g of spray-dried cells with a content of caffeic acid derivatives of 5.46 g were obtained from 5 liters of cell suspension. The obtained preparation represents the spray-dried stems of *Lippia citriodora.*

### EXAMPLE 4 ― DETERMINATION OF THE CONTENT OF CAFFEIC ACID DERIVATIVES IN CELL CULTURES SELECTED FOR THE PREPARATION OF STEMS

Determination of the content of caffeic acid derivatives in cell cultures selected for the preparation of stems was performed using 10 ml of cell suspension for the analysis. An equal volume of methanol and 5 mg/ml of ascorbic acid are added to the collected sample. The obtained suspension is homogenized using an ultraturrax.

After the homogenizing procedure, the suspension is filtered through a 0.45 µm Millipore filter or centrifuged. After appropriate dilution, an aliquot of the cleared solution is injected into an HPLC column for quantitative and qualitative analysis. This method provides data about production of caffeic acid derivatives per unit of volume (µg/ml of culture).

In order to obtain data about the specific production (µg/g of fresh cells) a known quantity of culture is collected and filtered through a Buchner funnel; the separated cells are weighed and then suspended in methanol with the addition of ascorbic acid and homogenized using an ultraturrax as described above. Similarly, the suspension is filtered or centrifuged and analyzed by HPLC. A reverse phase HPLC is used for the quantitative determination of the components of the caffeic acid derivative mixture. Analyte detection occurs through measurement of absorbance at 330 nm, which is the absorption maximum of caffeic acid. HPLC analyses were carried out on an Agilent liquid chromatographer 1100 equipped with a photodiode detector.

The used method is described below:
• Column: Phenomenex Luna C₁₈ 4.6 x 150 mm, thermoregulated at 30°C
• Phase A: 0.01N H₃PO₄ in H₂O
• Phase B: 0.01N H₃PO₄ in CH₃CN
• Flow rate: 0.8 ml/min
• Detection: 330 nm
• Eluition using the following gradient:

| **time** | **B (%)** |
|---|---|
| 0 | 0 |
| 10 | 10 |
| 15 | 20 |
| 20 | 25 |
| 25 | 35 |
| 30 | 45 |
| 35 | 55 |
| 40 | 0 |

Individual peaks were quantified in relation to caffeic acid.

The preparations obtained according to the processes described in examples 1, 2, and 3 were used to set up some biological assays. Hereinafter, some examples of antioxidant and anti-inflammatory activities, collagenase and hyaluronidase inhibition, stimulation of collagen production, etc., of the meristem cell preparations object of the present invention, are provided for the purposes of non-limiting illustration.

### EXAMPLE 5 ― DETERMINATION OF THE ANTIOXIDANT ACTIVITY OF GLYCEROLIC STEMS FROM BUDDLEJA DAVIDII

Determination of the antioxidant activity of glycerolic stems from a culture of *Buddleja davidii* (prepared as described in example 1) was performed using the DPPH free radical. 500 µl of a 100 µM DPPH solution was incubated for 15 minutes at room temperature with an equal volume of glycerolic stems from *Buddleja davidii.* During the incubation, DPPH is reduced and loses its characteristic color shifting from purple to pale yellow. Absorbance values were measured on a spectrophotometer at 515 nm.

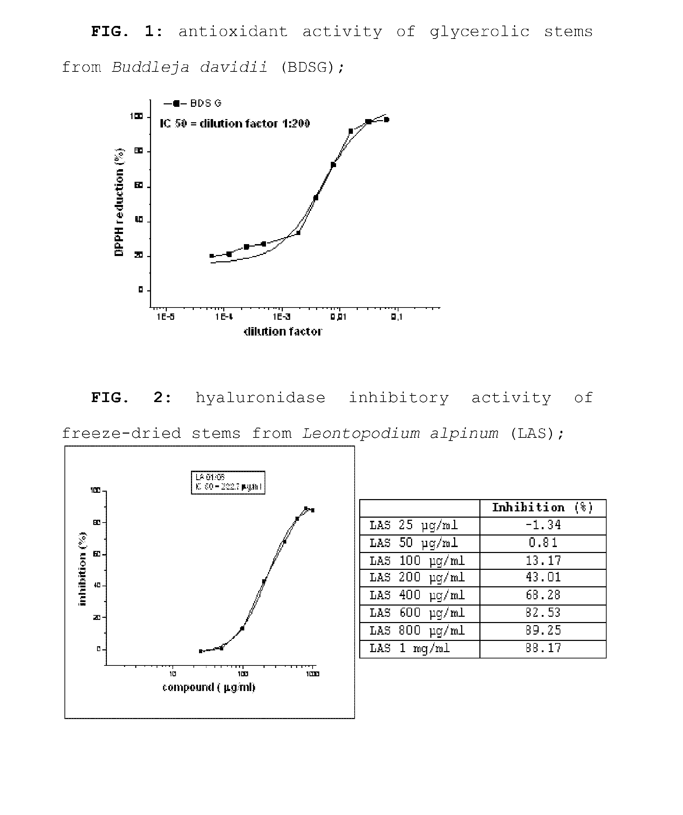

The results obtained from the assay on a sample of the glycerolic stem preparation from *Buddleja davidii* are reported in Fig. 1.

### EXAMPLE 6 ― DETERMINATION OF THE HYALURONIDASE INHIBITORY ACTIVITY OF FREEZE-DRIED STEMS FROM LEONTOPODIUM ALPINUM

The ability of freeze-dried stems from *Leontopodium alpinum* (prepared as described in example 2) to inhibit degradation of the hyaluronic acid present in the extracellular matrix was assessed using the hyaluronidase activity inhibition assay.

The assays envisages a first incubation of the sample to be analyzed in the presence of 50 U of the enzyme hyaluronidase for 15 minutes at 37°C, followed by a second incubation for 45 minutes at 37°C after the addition of a 3 mg/ml hyaluronic acid solution.

Samples were boiled for 3 minutes, cooled rapidly on ice, and added to 3 ml of a 10 mg/ml DMBA solution.

The solution was incubated for 20 minutes at 37°C and the absorbance values were measured on a spectrophotometer at 544 nm.

The results obtained from the assay on the sample of *Leontopodium alpinum* are reported in Fig. 2.

### EXAMPLE 7― DETERMINATION OF THE ANTI-INFLAMMATORY ACTIVITY OF SPRAY-DRIED STEMS FROM LIPPIA CITRIODORA

Determination of the anti-inflammatory activity of spray-dried stems from *Lippia citriodora IRB Lippia C19* (as described in example 3) was assessed by a biological assay using the mouse macrophage cell line RAW 264,7. The assay was set up by seeding cells of the RAW 264,7 cell line on 96-well plates in DMEM medium + 10% FCS. When cells reached 80% confluency, culture medium was replaced by medium conditioned with the compound to be tested in the presence or absence of a 20 µg/ml LPS solution and incubated for 24 hours at 37°C in a humidified 5% CO₂ atmosphere. At the end of the incubation period, 90 µl of culture supernatant was mixed with 10 µl of Griess reagent and left at room temperature for 10 minutes. Absorbance of the solution was measured on a spectrophotometer at 540 nm and the sample NO concentration was extrapolated from a NaNO₂ calibration curve.

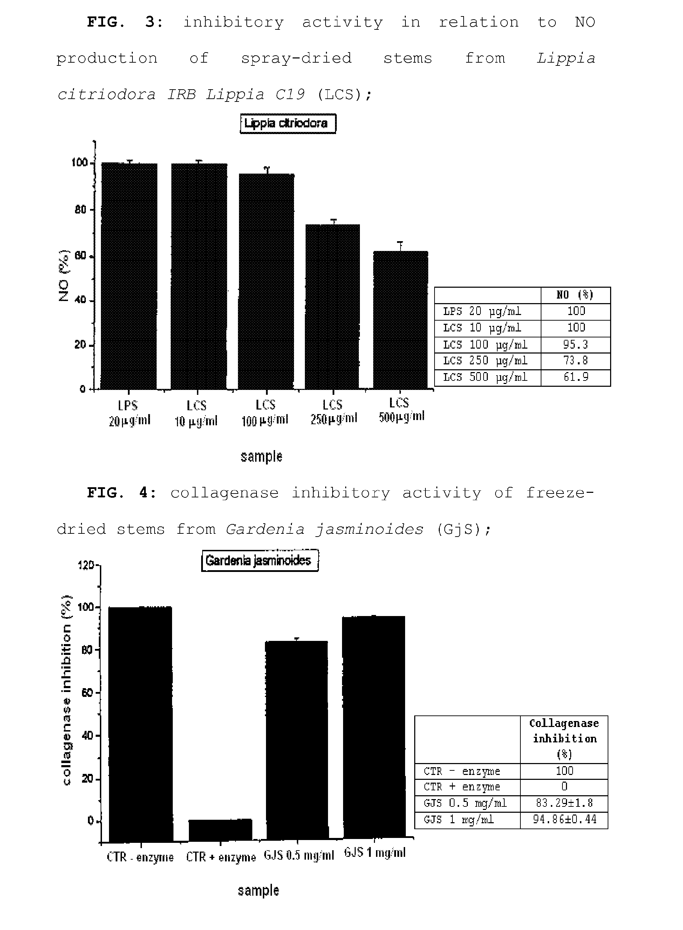

The results obtained from the assay on the spray-dried stems from *Lippia citriodora IRB lippia C19* are reported in Fig. 3.

### EXAMPLE 8― DETERMINATION OF THE COLLAGENASE INHIBITORY ACTIVITY OF FREEZE-DRIED STEMS FROM GARDENIA JASMINOIDES

Determination of the inhibitory activity of freeze dried stems from *Gardenia jasminoides* in relation to degradation of the extracellular matrix collagen through collagenase inhibition was assessed by spectrophotometric measurement of hydroxyproline. The assays consists in the gelification of 500 µl of collagen in 6-well plates and in the subsequent incubation of the stem sample in the presence or absence of collagenase 70 U/ml for 15 hours at 37°C. After the incubation period, samples are washed with 1x PBS, dried in nitrogen gas, and subjected to acidic hydrolysis with 6M HC1 for 15-18 hours at 110°C. After another drying cycle in nitrogen gas and resuspension in acetate-citrate buffer, 100 µl of sample is incubated at room temperature in the dark for 25 minutes with 100 µl of a 7% chloramine-T solution. After addition of 100 µl of Ehlrich's solution, samples are subjected to a second incubation at 60°C for 15 minutes, after which samples are read on a spectrophotometer at 540-560 nm and the quantity of hydroxyproline is extrapolated from a calibration curve.

The results obtained from the assay on a sample of freeze-dried stems from *Gardenia jasminoides* at a concentration of 0.5 -1- mg/ml are reported in Fig. 4.

### EXAMPLE 9 ― ETERMINATION OF THE COLLAGEN STIMULATORY ACTIVITY OF FREEZE-DRIED STEMS FROM ECHINACEA ANGUSTIFOLIA

Assessment of the ability of freeze-dried stems from *Echinacea angustifolia IRBEA66* ((Deutsche Sammlung von Mikroorganismen und Zellkulturen, Braunschweig, D: DSMZ 17522, date of deposit: May 18^{th}, 2005) to stimulate collagen production was measured with a biological assay using the human fibroblast cell line HFF1.

The assay was set up by seeding HFF1 cells in 12-well plates in DMEM medium + 10% FCS.

When cells reach 80% confluency, the culture medium is replaced by medium conditioned in the presence and absence of the compound to be tested and incubated for 72 hours at 37°C in a humidified 5% CO₂ atmosphere. At the end of the incubation, cells are washed with 1x PBS and fixed with a solution of Buoin's fluid for 1 hour at room temperature.

After removing the fixing solution and washing the cells twice with water, cells are stained by addition of Sirius Red for 1 hour under constant agitation.

At the end of the incubation, the staining solution is discarded, the sample is washed twice with water, and 0.1M NaOH is added for 30 minutes at room temperature.

Absorbance of the solution is measured at 550 nm and the concentration of the collagen present in the samples is extrapolated from a calibration curve of collagen and normalized against the total protein content.

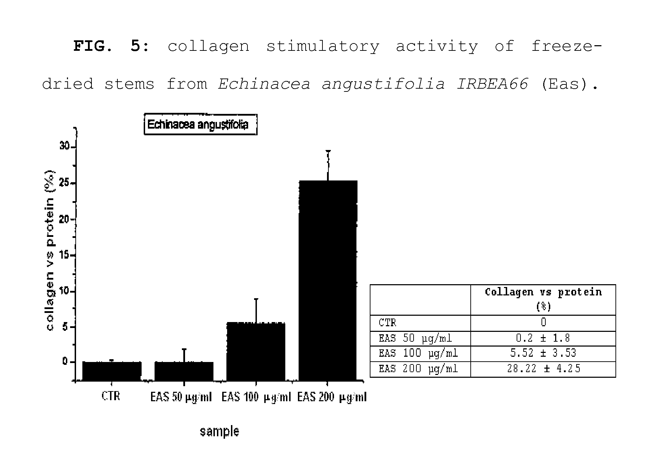

The results obtained from the assay on a sample of *Echinacea angustifolia IRB EA66* at concentrations of 50 - 100 - 200 µg/ml are reported in Fig. 5.

The preparations of the invention can be advantageously used for the following biological activities which they contain:
- antioxidant activity, in the prevention and treatment of disorders related to free radical-induced damage and with inflammatory components: cutaneous disorders such as seborrhoeic, atopic, and irritative dermatitis, cellulitis, mucosal diseases comprising oropharyngeal, gastrointestinal, and vaginal mucosa, dental disorders, disorders of the respiratory apparatus, articular and ophthalmic pathologies, and reduced immune response;
- anti-inflammatory activity, in the prevention and treatment of alterations related to chronic and acute inflammatory states;
- cytoprotective activity, in the prevention of damages caused by environmental stress;
- collagenase inhibitory activity and collagen production stimulatory activity, in the prevention and treatment of cutaneous disorders and in the delay of cutaneous aging processes;
- hyaluronidase inhibitory activity, for the maintenance of skin tone, hydration, and to prevent and fight cutaneous relaxation.

In order to strengthen, modulate, synergize, and widen the range of biological activities mentioned above, mixtures of one or more praparations of the invention can be prepared in preparation ratios ranging from 1:10 to 10:1, based on the necessities and biological activities that it is desired to maximize.

Hence, preparation compositions represent a further object of the invention that can be obtained from a preparation as defined above or from a mixture of one or more preparations as defined above.

Doses, times, and routes of administration of the treatment will be selected on the basis of the type, stage, severity, and location of manifestation of the pathology. For all the pathologies mentioned above, oral administration is indicated, but also topical and transdermal, and in any case so as to make active ingredients maximally available. For oral formulations, administrations in the form of tablets, lozenges, and capsules, but also powders and suspensions, are preferred; for topical treatment, gels, creams, ointments, and solutions compatible with dermal and mucosal use are preferred, in addition to eye washes for administration into the conjunctival sac.

The composition therapeutic dose varies between 0.1 mg and 2 g per day and preferably between 5 and 150 mg per day, taken in a single dose or in 2-4 doses or in slow release forms, depending on the patient's therapeutic needs, and for periods of time varying between 1 and 120 days.

At appropriate concentrations, the same compositions may be formulated in the form of supplements, to be taken orally for prevention or as adjuvant treatments of pathologies resulting from disreactive states in humans or in veterinary medicine. At appropriate concentrations and in appropriate formulations, the compositions of the invention may be likewise used in cosmetics and tricological treatments.

## Claims

1. Preparation of meristem cells with a high content of caffeic acid derivatives, said cells being derived from cell cultures of plants belonging to the genera *Achillea, Ajuga, Buddleja, Centella, Cynaris, Echinacea, Forsythia, Gardenia, Glycyrrhiza, Hippeastrum, Hoodia, Lamium, Leontopodium, Lippia, Lonicera, Magnolia, Marrubium, Passiflora, Phytolacca, Plantago, Ruta, Stachys, Stapelia, Syringa, Teucriun,* or *Zanthoxylum.*

2. Preparation of meristem cells according to claim 1, in which said plants belong to the species *Achillea millefolium, Ajuga reptans, Buddleja davidii, Centella asiatica, Cynaris scolimus, Echinacea angustifolia, Echinacea purpurea, Echinacea pallida, Forsythia viridissima, Gardenia jasminoides, Glycyrrhiza glabra, Glycyrrhiza echinata, Hippeastrum lady jane, Hoodia gordonii, Lamium album, Leontopodium alpinun, Lippia citriodora, Lonicera japonica, Magnolia grandiflora, Magnolia solangeniana, Marrubium vulgare, Passiflora palmeri, Passiflora quadrangularis, Phytolacca americana, Plantago lanceolata, Ruta patavina, Scrophularia nodosa, Stachys officinalis, Stapelia grandiflora, Syringa vulgaris, Teucriun chanaedrys,* or *Zanthoxylum americanum.*

3. Preparation of meristem cells according to claim 1 or 2, in which said preparation is a solution or suspension of said cells in glycerol or in glycerol with the addition of xanthan gum in a quantity from 0.1 to 2% w/w; or a freeze-dried preparation of said cells, or a spray-dried preparation of said cells.

4. Preparation according to claim 3, in which said solution or suspension of meristem cells in glycerol is in a weight/volume ratio ranging from 5:95 to 50:50, or about 20:80.

5. Preparation according to any of the claims from 1 to 4, in which said preparation contains a quantity of caffeic acid derivatives greater than 1% by weight, as expressed by the weight of fresh cells, or greater than 1% by weight, or greater than 5% by weight.

6. Preparation according to any of the claims from 1 to 5, for medical use on humans or animals.

7. Preparation according to claim 6, for use as antioxidant in the prevention and treatment of disorders related to damage induced by free radicals and with inflammatory components: cutaneous disorders like seborrhoeic, atopic, and irritative dermatitis, cellulitis, mucosal disorders including oropharyngeal, gastrointestinal, and vaginal mucosa, dental pathologies, disorders of the respiratory apparatus, articular and ophthalmic pathologies, and reduced immune response.

8. Preparation according to claim 6, for use as anti-inflammatory in the prevention and treatment of disorders related to chronic and acute inflammation.

9. Preparation according to claim 6, for use as a cytoprotective agent in the prevention of damages caused by environmental stress.

10. Preparation according to claim 6, for use as collagenase inhibitor and collagen production inducer in the prevention and treatment of cutaneous disorders and for the delay of cutaneous aging processes.

11. Preparation according to claim 6, for use as hyaluronidase inhibitor for the maintenance of skin tone, hydration, and to prevent and fight cutaneous relaxation.

12. Human or veterinary pharmaceutical composition comprising a preparation as defined in any of the claims from 1 to 5, together with pharmacologically acceptable eccipients.

13. Pharmaceutical composition according to claim 12 for oral, topic, or transdermal administration.

14. Pharmaceutical composition according to claim 13, in which said oral formulation is in the form of tablets, lozenges, capsules, powders, or suspensions; said topic formulation is in the form of gels, creams, or ointments, solutions compatible with dermal and mucosal use, eye washes for administration into the conjunctival sac.

15. Cosmetic composition comprising a preparation as defined in any of the claims from 1 to 5, together with cosmetic eccipients.

16. Cosmetic composition according to claim 15, said composition being a tricologic composition.

17. Human or animal supplement comprising a preparation as defined in any of the claims from 1 to 5.

18. Pharmaceutical or cosmetic composition or supplement according to any of the claims from 12 to 17, comprising preparations obtained from one or more plants of different genera or species selected from those defined in the previous claims, in a preparation ratio ranging from 1:10 to 10:1.

19. Process for producing a preparation according to any of the claims from 1 to 5, comprising the following stages:
1) selection of clones deriving from cell cultures of plants belonging to the genera *Achillea, Ajuga, Buddleja, Centella, Cynaris, Echinacea, Forsythia, Gardenia, Glycyrrhiza, Hippeastrum, Hoodia, Lamium, Leontopodium, Lippia, Lonicera, Magnolia, Marrubium, Passiflora, Phytolacca, Plantago, Ruta, Stachys, Stapelia, Syringa, Teucriun,* and *Zanthoxylum,* in which the quantities of the caffeic acid derivatives are the greatest;
2) recovery of the plant biomass deriving from a cell culture of said clones selected according to stage 1) on liquid culture medium;
3) separation of cells from the culture medium in order to give a preparation of meristem cells.

20. Process according to claim 19, further comprising a solubilization or suspension stage of said cells in glycerol or in glycerol with the addition of xanthan gum in a quantity from 0.1 to 2% w/w, or in a quantity of about 0.3% w/w, or in buthylenglycol; or a freeze-drying stage of said cells; or a spray-drying stage of said cells.
